# EUROPEAN PATENT SPECIFICATION

(11) **EP 1 675 481 B1**
(45) Date of publication and mention of the grant of the patent: **19.11.2008**
(21) Application number: 04793673.7
(22) Date of filing: 25.10.2004
(51) Int. Cl.: A23L 1/29, A23L 1/30, A61K 31/733, A61K 31/702, A61K 35/74, A61P 37/00

(54) **SYNBIOTIC COMPOSITION FOR INFANTS**
SYMBIOTISCHE ZUSAMMENSETZUNG FÜR KLEINKINDER
COMPOSITION SYMBIOTIQUE POUR NOURRISSONS

(30) Priority: 24.10.2003 EP 03078374
(43) Date of publication of application: 05.07.2006
(73) Proprietor: N.V. Nutricia, 2712 HM Zoetermeer (NL)
(72) Inventor: SPEELMANS, Gelske, NL-6708 LW Wageningen (NL); KNOL, Jan, NL-6708 SM Wageningen (NL); HAARMAN, Monique, NL-6702 BW Wageningen (NL); GARSSEN, Johan, NL-3434 SG Nieuwegein (NL)
(74) Representative: van Westenbrugge, Andries
(86) International application number: PCT/NL2004/000748
(87) International publication number: WO 2005/039319

(56) References cited:
- EP-A- 0 856 259
- EP-A- 0 904 784
- EP-A- 1 175 905
- WO-A-03/013559
- DE-A- 10 008 279
- DE-A- 10 206 995
- DE-A- 19 836 339
- US-A1- 2002 015 990
- US-A1- 2003 031 659
- PATENT ABSTRACTS OF JAPAN vol. 1999, no. 02, 26 February 1999 (1999-02-26) & JP 10 309178 A (WAKAMOTO PHARMACEUT CO LTD), 24 November 1998 (1998-11-24)
- FAMULARO G ET AL: "TRADITIONAL AND HIGH POTENCY PROBIOTIC PREPARATIONS FOR ORAL BACTERIOTHERAPY" BIODRUGS, AUCKLAND, NZ, vol. 12, no. 6, 1999, pages 455-470, XP009001219 ISSN: 1173-8804
- MORO G ET AL: "Dosage-Related Bifidogenic Effects of Galacto- and Fructooligosaccharides in Formula-Fed Term Infants" JOURNAL OF PEDIATRIC GASTROENTEROLOGY AND NUTRITION, RAVEN PRESS, NEW YORK, NY, US, vol. 34, no. 3, March 2002 (2002-03), pages 291-295, XP008029577 ISSN: 0277-2116 cited in the application
- MORISHITA Y ET AL: "Galactooligosaccharide in combination with Bifidobacterium and Bacteroides affects the population of Clostridium perfringens in the intestine of gnotobiotic mice." NUTRITION RESEARCH, vol. 22, no. 11, November 2002 (2002-11), pages 1333-1341, XP002295913 ISSN: 0271-5317
- ISOLAURI E ET AL: "PROBIOTICS IN THE MANAGEMENT OF ATOPIC ECZEMA" CLINICAL AND EXPERIMENTAL ALLERGY, BLACKWELL SCIENTIFIC PUBLICATIONS, LONDON, GB, vol. 30, November 2000 (2000-11), pages 1604-1610, XP001064231 ISSN: 0954-7894
- ISOLAURI E ET AL: "PROBIOTICS: EFFECTS ON IMMUNITY" AMERICAN JOURNAL OF CLINICAL NUTRITION, BETHESDA,MD, US, vol. 73, no. SUPPL, February 2001 (2001-02), pages 444S-450S, XP000984966 ISSN: 0002-9165
- KANAMORI YUTAKA ET AL: "Combination therapy with Bifidobacterium breve, Lactobacillus casei, and galactooligosaccharides dramatically improved the intestinal function in a girl with short bowel syndrome: A novel synbiotics therapy for intestinal failure" DIGESTIVE DISEASES AND SCIENCES, vol. 46, no. 9, September 2001 (2001-09), pages 2010-2016, XP009036227 ISSN: 0163-2116
- LAMOUREUX L ET AL: "PRODUCTION OF OLIGOSACCHARIDES IN YOGHURT CONTAINING BIFIDOBACTERIA AND YOGURT CULTURES" JOURNAL OF DAIRY SCIENCE, AMERICAN DAIRY SCIENCE ASSOCIATION. CHAMPAIGN, ILLINOIS, US, vol. 85, no. 5, May 2002 (2002-05), pages 1058-1069, XP001124200 ISSN: 0022-0302

## Description

### TECHNICAL FIELD OF THE INVENTION

The present invention relates to preparations comprising a probiotic and a prebiotic for infants, in particular for non-breast-fed infants.

### BACKGROUND OF THE INVENTION

Infants are devoid of intestinal flora at birth. As a result of contact with the mother during birth and subsequent breast feeding, the intestinal flora rapidly develops and increases. During the development, the intestinal flora is still immature and its equilibrium is fragile and quickly prone to changes and thus to the occurrence of diseases and affections in the presence of pathogens. Breast-fed infants are known to be less afflicted by infections or diseases than non-breast-fed infants. Hence, breast-fed babies have less gastro-intestinal infections in terms of both incidence and duration, less atopic diseases such as allergy, eczema, allergy induced asthma, and less constipation than non-breast-fed infants.

Generally, the intestinal flora of breast-fed infants is primarily composed of bifidobacteria and lactic acid bacteria. Breast milk contains human milk oligosaccharides (HMO), which are a growth factor for bifidobacteria in the intestine of infants. The flora of formula-fed infants is more diverse and contains in general more *Bacteroides, Clostridium* and

*Enterobacteriaceae* species. Formula-fed infants have about one-tenth to roughly two-third the number of bifidobacteria of breast-fed infants. Bifidobacteria are considered to be important in maintaining a well-balanced intestinal microbiota and it has been postulated that bifidobacteria have several health-promoting effects, including the prevention and/or treatment of diarrhea and intestinal infections. Furthermore, bifidobacteria have been shown to play a role in the immune system of the host.

The intestinal flora of infants may be modified by nutritional changes in the diet, like consumption of probiotics or prebiotics. As an example of the probiotics approach, EP-A-0,904,784 describes the administration of a mixture of micro-organism strains, including *Bifidobacterium* strains. However, a problem associated therewith is that the mixture of microbes, while providing some health benefit, may also have a deleterious effect on the still immature intestinal flora of non-breast-fed infants due to its broad spectrum of action. Further, many probiotic supplements have a short shelf-life and contain too low a number of living microorganisms, thereby failing to provide the expected probiotic effects.

Prebiotics are defined as non-digestible food ingredients that selectively stimulate the growth and/or activity of one or more bacteria in the colon and thereby beneficially affect the host (Gibson and Roberfroid, J. Nutr. 125:1401-14121995). A preferable way to improve the intestinal flora of bottle-fed babies is to selectively stimulate the bifidobacteria already present in the bottle-fed infant's intestine by specific non-digestible oligosaccharides, i.e. prebiotics. Also, mixtures of oligosaccharides and polysaccharides have been proposed as prebiotics, e.g. in WO 00/08948 DE 19836339. One example is the combination of galacto-oligosaccharide with fructopolysaccharides. The bifidobacteria level in infants receiving a formula containing these prebiotics has been shown to be elevated in comparison with a standard formula (see e.g. Moro et. al. J. Pediatr. Gastroenterol. Nutr. 34:291-295, 2002).

The approach up to now was to promote bifidobacteria in general, i.e. on the genus level. The genus *Bifidobacterium* consists of many different species, which differ in metabolism, enzyme activity, oligo- and polysaccharide utilisation, cell wall composition, and interaction with the host's immune system. It therefore can be expected that not every species of *Bifidobacterium* has the same functional effect on the infant. Examples of different *Bifidobacterium* species are *B. longum, B. breve, B. infantis, B. adolescentis, B. bifidum, B. animalis*, and *B. dentium. B. adolescentis* is more prevalent in the flora of adults, and is less common in faeces of healthy infants and babies. *B. animals* /*B. lactis* is not naturally occurring in humans, and *B. dentium* is a pathogenic bacterium. In healthy infants the bifidobacterial flora is mainly composed of *Bifidobacterium infantis, B. breve* and *B. longum*. Kalliomaki et. al. (Curr Opin Allergy Clin Immunol. 2003 Feb;3(1):15-20, and references cited therein), reported that allergic infants harbour an adult-like *Bifidobacterium* flora whereas a typical infant *Bifidobacterium* flora was shown in healthy infants, indicating a correlation between the occurrence of certain *Bifidobacterium* species and the chance of developing allergy. These results indicate that the stimulation of the genus *Bifidobacterium* in the baby's colon may not be sufficient. It is the aim to achieve a flora in bottle-fed infants that is reminiscent to the flora of breast fed babies on a species level.

For the purpose of the present invention, "breast-fed infants" refers to infants which are exclusively fed with human breast milk. "Non- or partially breast-fed infants" means infants which are not or not exclusively receiving human breast milk. This definition includes those infants which are receiving at least the content of a bottle per day, i.e. at least 80 ml of formula milk per day, the rest, if any, of the nutrition being provided from solid nutrition or liquid nutrition such as breast milk, i.e. partly-breast-fed infants.

### SUMMARY OF THE INVENTION

It was been found that the increase in the level of *Bifidobacterium* using mixtures of non-digestible carbohydrates also regulates the *Bifidobacterium* population to a more infant-like population, i.e. low in *B. catenulatum, B. pseudocatenulatum* and *B. adolescentis*, whereas infants fed with a standard formula exhibit a more adult-like flora, that is more predominant in *B. catenulatum, B. pseudocatenulatum* and *B. adolescentis.* It was also found that the *Bifidobacterium* population in such prebiotic-fed infants was still deficient in one particular microorganism, namely *Bifidobacterium breve.*

Accordingly, in one aspect of the invention, there is provided a preparation comprising *Bifidobacterium breve* and a mixture of non-digestible carbohydrate prebiotics. It was found that such a preparation is beneficial and very suitable for regulating the *Bifidibacterium* population on a species level in the gastro-intestinal tract of infants. Furthermore,it was surprisingly found that addition of other *Bifidobacterium* species than *B. breve* species is not necessary, as they are sufficiently regulated by the preparation as such.

In another aspect of the invention, there is provided a preparation comprising *Bifidobacterium breve* and a mixture of non-digestible carbohydrate prebiotics, wherein the mixture of non-digestible carbohydrate contains at least two different, substantially soluble carbohydrate components A and B.

In another aspect of the invention, there is provided the use of the preparation for non- or partially breast-fed infants.

In a further aspect of the invention, there is provided the use of the preparation for the manufacture of a composition for the regulation of the *Bifidobacterium* species population in the gastro-intestinal tract of non- or partially breast-fed infants.

In a further aspect of the invention, there is provided the use of the preparation for the manufacture of a composition for the prevention or treatment of an immune condition.

In a further aspect of the invention, there is provided the use of a carbohydrate mixture for regulating the population of *Bifidobacterium catenulatum, B. pseudocatenulatum* and/or *Bifidobacterium adolescentis* in the gastro-intestinal tract of non- or partially breast-fed infants.

### DETAILED DESCRIPTION OF THE INVENTION

### Preparation

### 1) Bifidobacterium breve

*Bifidobacterium breve* is an essential ingredient of the present invention. This bacterium has been found by the Applicant's method of detection as being present in limited quantities in non-breast-fed infants. Accordingly, the administration of this bacterium with the carbohydrate mixture enables the normalisation of the *Bifidobacterium* species population to a level equivalent to that present in the gastrointestinal tract of breast-fed infants.

Preferred *Bifidobacterium breve* strains are those selected from isolates from the faeces of healthy breast-fed infants. Typically, these are commercially available from producers of lactic acid bacteria, but they can also be directly isolated from faeces, identified, characterised and produced. Examples of commercially available *B. breve* are *B. breve* Bb-03 from Rhodia, *B. breve* MV-16 from Morinaga, and *B. breve* from Institut Rosell, Lallemand, but *B. breve* can also be obtained from culture collections such as DSM 20091, and LMG 11613.

The amount of *B. breve* in the preparation of the invention can be based on the total amount of soluble non-digestible carbohydrates, and is preferably from 10⁷ to 10¹¹, more preferably from 10⁸ to 10¹⁰ cfu of the bacteria per g of the total of these carbohydrates.When the preparation is used as a supplement, the *Bifidobacterium breve* is most preferably present in the supplement in an amount of from 1x10⁶ to 1.5x10¹¹ cfu/g, preferably from 3x10⁷ to 5x10¹⁰ cfu/g, more preferably from 5x10⁸ to 1x10¹⁰ cfu/g. When the preparation is used as a (complete) infant nutrition, the *B. breve* is most preferably present in the nutrition in an amount of from 1x10⁴ to 1x10¹⁰ cfu/g, preferably from 5x10⁶ to 3x10⁹ cfu/g, more preferably from 1x10⁷ to 5x10⁸ cfu per g of the infant nutrition. These concentration are chosen in such a way that the daily dose is about 1x10⁶ to 1.5x10¹¹ cfu/g, preferably from 3x10⁷ to 5x10¹⁰ cfu/g, more preferably from 5x10⁸ to 1x10¹⁰ cfu/g.

### 2) Mixture of non-digestible carbohydrate prebiotics

A mixture of non-digestible carbohydrate prebiotics is also an essential element of the invention. By "non-digestible", it is meant that that the carbohydrates remain undigested in the gastrointestinal tract and reach the large intestine unresorbed.

For the purpose of the invention, the mixture of non-digestible carbohydrates contains at least two different, essentially soluble carbohydrate components A and B, which remain undigested in the gastrointestinal tract and reach the large intestine unresorbed. The carbohydrate mixtures according to the present invention may also consist exclusively of these two carbohydrate components A and B.

In the mixture of at least two non-digestible soluble carbohydrate components A and B, the carbohydrate component A is present in an amount of from 5 to 95 % by weight of the sum of carbohydrate components A and B. Furthermore, at least 50%, preferably at least 75%, of the total non-digestible soluble carbohydrates of components A and B is selected from disaccharides to eicosasaccharides (polysaccharides having 20 monosaccharide units); the remainder may be non-digestible monosaccharides and non-digestible polysaccharides which are longer than 20 units. It is also preferred that more than 95%, preferably more than 98% of the total soluble non-digestible carbohydrates has a chain length of no more than 100 units. Where percentages and averages are mentioned in this description, percentages and averages by weight are meant, unless it is evident that another basis is meant or when otherwise specified.

The carbohydrates of components may differ in:
(i) in the (average) number of monosaccharide units of the carbohydrate, component A having an average chain length which is at least 5 monosaccharide units lower than the average chain length of component B; this means that if the carbohydrates of A and B have the same structural units, i.e. they form a mixture of homologues differing only in chain length, the distribution of the homologues must have two maximums, one maximum being below 7, and one above 7, the two maximums being at least 5 units apart; the carbohydrates up to 6 units (hexasaccharides) are then part of component A, and the carbohydrates from 7 units (heptasaccharides) onwards are part of component B;
(ii) in the structure of the monosaccharide units of the carbohydrate, component A being built up from different structural units from component B; where A and/or B are built up from repeating combinations of different monosaccharides units, for example in the case of galactomannans and arabinogalactans, at least 50% of the monosaccharide units of the two components should be different (in the above example either or both should have less than 50% anhydrogalactose units);

Preferably, component A is selected from indigestible monosaccharides up to hexasaccharides of the same carbohydrate structure, and component B is selected from heptasaccharides and higher polysaccharides of the same carbohydrate structure. Carbohydrate component A thereby consists of at least one non-digestible monosaccharide or at least one non-digestible oligosaccharide. With oligosaccharides it is understood those comprising 2 up to and including 6 monosaccharide units. Carbohydrate component A may also, and preferably, be formed by a mixture of two or more of the mentioned saccharides. It may therefore be comprised of any number of various monosaccharides and/or oligosaccharides of that kind, i.e. of the same structure.

According to this preferred embodiment, carbohydrate component B consists of at least one polysaccharide comprising 7 or more monosaccharide units. With polysaccharides it is understood those starting from heptasaccharide (e.g. heptasaccharide, octasaccharide, nonasaccharide, decasaccharide, etc.). There is no specific upper limit to the chain length of polysaccharides, and they may be as long as several hundreds or even thousands of monosaccharide units. However, chain lengths of more than 100 (about 16 kD), and especially those of more than 700 (about 100 kD) are less preferred according to the invention. Preferably, component B does not contain more than 5% or even not more than 2% of homologues having more than 100 monosaccharide units. Carbohydrate component B may also be comprised of only one polysaccharide of that kind or, preferably, of two or more polysaccharides of different length of that kind, i.e. of the same structure.

Carbohydrate component A represents up to 95 wt% of the sum of carbohydrate component A and carbohydrate component B (A + B = 100 wt%). Carbohydrate component B represents 5 to 95 wt% of the sum of carbohydrate component A and carbohydrate component B. According to a preferred embodiment, component A constitutes 95 to 60 wt%, more preferably 95 to 80 wt.% and in particular 95 to 90 wt%, and component B 5 to 40 wt%, more preferably 5 to 20 wt.% and in particular 5 to 10 wt% of the carbohydrates *present in toto*, with A + B = 100 wt%.

As soluble carbohydrates in the sense of the present invention are understood those that are at least 50% soluble, according to a method described by L. Prosky et al, J. Assoc. Anal. Chem 71: 1017-1023, 1988.

At least 80 wt% of the carbohydrates or saccharides out of the sum of carbohydrate component A and B thereby have a prebiotic effect. Preferably, at least 80 wt% of the carbohydrates belonging to carbohydrate component A, and also at least 80 wt% of those belonging to carbohydrate component B, have a prebiotic effect. In other words, preferably at least 80 wt% each of the carbohydrates or saccharides out of carbohydrate components A and B, are intended to reach the large intestine in an undigested (hence not resorbable in the small intestine) manner. In other words, these carbohydrates or saccharides of carbohydrate components A and B in the gastrointestinal tract are neither resorbed and digested in the stomach nor in the small intestine, but reach the large intestine as such.

With a prebiotically active carbohydrate according to the present invention it is understood a carbohydrate, which reaches the large intestine undigested (hence not resorbable in the small intestine), and there, it selectively encourages the growth and/or the activity of one or of a restricted number of bacterial species in the intestine, and consequently promotes health. This prebiotic effect of such carbohydrates and their specific mechanisms are described in detail in "G.F. Gibson & M.B. Roberfroid, J.Nutr. 1995; 125: 1401-1412", whereto explicit reference is made herewith, and of which the disclosure is included in the present document.

The proportion of the non-prebiotically active carbohydrates or saccharides of carbohydrate components A and B therewith amounts to a maximum of 20 wt%. These carbohydrates or saccharides refer to those which are actually soluble but can be excreted in an undigested form. These carbohydrates can exercise a physical effect in that they increase, for example, the volume of the faeces or prompt a water adsorption.

For the assessment of the proportion determining the carbohydrate components A and B in a dietary or pharmaceutical product, the following steps are carried out. In a first stage, all soluble carbohydrates are extracted from the product by means of water. Fats and proteins are removed from the extract. In a second stage, the soluble carbohydrates or the extract, respectively, are digested by means of human enzymes, e.g. human amylase, human pancreatic juice or small intestine ciliated border preparations. The yield of non-digested carbohydrates (except for the *in vivo* resorbable monosaccharides obtained in this *in vitro* experiment), constitutes the two carbohydrate components A and B. 80 % thereof are supposed to be prebiotically active.

Hence, the carbohydrate mixtures to be used in the preparation of the invention are those, wherein the carbohydrates, which are soluble and undigested in the sense described above, fulfil the herein specified criteria and constitute the carbohydrate components A and B.

Carbohydrate component A may, for example, consist of one or more of the following carbohydrates: β-galacto-oligosaccharides, α-galacto-oligosaccharides, fructo-oligosaccharides, inulo-oligosaccharides, fuco-oligosaccharides, manno-oligosaccharides, xylo-oligosaccharides, sialyl-oligosaccharides, N-glycoprotein oligosaccharides, O-glycoprotein oligosaccharides, glycolipid oligosaccharides, cello-oligosaccharides, chitosan-oligosaccharides, chitin-oligosaccharides, galacturono-oligosaccharides, glucurono-oligosaccharides, β-glucan (e.g. 1,3-) oligosaccharides, arabinoxylo-oligosaccharides, arabino-galacto-oligosaccharides, xylogluco-oligosaccharides, galactomanno-oligosaccharides, rhamno-oligosaccharides, soy oligosaccharides (stachyose, raffinose, verbascose), and lacto-N-neotetraose. or Carbohydrate component B may, for example, be formed of one or more of the following carbohydrates or saccharides: fruct(os)anes including inulins, galactans, fucoidans, arabinans, xylans, xanthans, β-glucans, indigestible polydextrose, indigestible maltodextrin, galacturonans, N-glycans, O-glycans, hyaluronic acids, chondroitins, xyloglucans, arabinogalactans, arabic gum, alginates, carrageenanes, galactomannans, glucomannans, arabinoxylanes, glycolipid glycans, glycoprotein glycans, proteoglycans, soy polysaccharides.It is to be noted that digestible carbohydrates are not part of the components A and B. Thus, glucose, fructose, galactose, sucrose, lactose, maltose and the maltodextrins do not count in these components, even if they are the lower homologues of e.g. galacto-oligosaccharides, fructo-oligosaccharides (inulin) and the like. Non-digestible carbohydrates of the invention, as a rule, do not have a large proportion of glucose units linked at the alpha 1,4 and/or alpha 1,6 position as in starch derivatives, as such carbohydrates will be digestible. However, certain starch-type polysaccharides and maltodextrins have been made indigestible or "resistant" by physical or enzymatic means; such oligo- and polysaccharides are included according to the invention, as long as they are sufficiently soluble.

By means of a selective combination of oligosaccharides and polysaccharides, and consequently the simultaneous presence of carbohydrate components A and B, the health-promoting microorganisms in the large intestine may be promoted and/or pathogenic microorganisms may be suppressed by an essentially higher efficiency than would be the case with only one of said carbohydrate components. Thus, it is possible with the administration of the carbohydrate combination, to achieve a very rapid restitution of a normal large intestinal flora, to maintain the same or to prophylactically prevent an alteration of the intestinal flora during situations of stress, and thus to influence the bacterial colonisation of the large intestine in a way, which is more efficient than the one with the previously used carbohydrates.

According to a preferred embodiment, at least 80 wt% of carbohydrate component A as well as of carbohydrate component B consist of carbohydrates, which are bifidogenic and/or which promote lactic acid bacteria. Due to such a combination of oligosaccharides and polysaccharides having said properties, the growth of the lactic acid bacteria may surprisingly be promoted in an essentially stronger manner than would be the case with oligosaccharides or polysaccharides alone. Not only lactic acid bacteria are thereby promoted, which are naturally present in the intestine, but also the growth of those is promoted - optionally even in a selective manner - which are introduced exogenously.

Apart from this indirect action via the bacteria themselves and their metabolites such as organic acids (acetate, lactate, etc.), pH effects and stimulation of colonozytes, direct physical effects such as peristalsis, water content, quantity of faeces, mechanical action upon the intestinal *mucosa*, are likewise positively influenced.

Thus, the carbohydrate mixtures dispose not only of a nutritive effect but also of a wide spectrum of activities. In addition to the above-described biological effects, the following may also be achieved by means of the inventive mixtures: stabilisation of natural microflora, prevention of pathogenic substances/organisms such as toxins, viruses, bacteria, fungi, transformed cells and parasites from adhering, dissolution of complexes of toxins, viruses, bacteria, fungi and other pathogens having endogenous cells, as well as their elimination from the body, and an acceleration of wound healing.

Thus, the mixtures are suitable for the prophylaxis and/or the treatment of symptoms or diseases occurring in conjunction with a disturbed intestinal flora, for example, as a consequence of the association or adhesion of the mentioned substances and organisms with or on epithelia or other endogenous cells.

Carbohydrate mixtures have found to be particularly efficient, when the carbohydrate components A have a different structure than the carbohydrate components B. This different structure may, for example, concern the monosaccharide composition when, for example, fructans are used on the one hand, and galactans on the other hand. This different structure may likewise concern the glycosidic bonding (e.g. α-galacto oligosaccharides versus β-galacto oligosaccharides or α-glucans (starch) versus β-glucans (cellulose)). The monomer composition, as well as the glycoside bonding may have an influence on the chemical behaviour (e.g. solubility) or on the physiological behaviour (e.g. digestibility).

Carbohydrates of the same structure are understood to be homologues which may differ in chain length, but which are composed of the same monosaccharides unit or combination of monosaccharide units. In general, the next homologue will differ from the previous one by the addition of one of the monosaccharide units as present in the previous one. Nevertheless, a single unit, usually a terminal one, may be different, as for example in certain fructans, which contain a chain of (anhydro)fructose units terminated with a glucose unit.

It is preferred that the chain length of the polysaccharide of component B, or the weight-average chain length in case of a mixture of polysaccharides, is at least three units, preferably at least five units longer than the chain length of the oligosaccharide of component A or the weight-average of a mixture of oligosaccharides. Preferably, the average chain length of the oligosaccharides A is between 2 and 6 units, and the average chain length of the polysaccharides B is between 7 and 30, more preferably between 8 and 20. Where both oligosaccharides and polysaccharides of the same structure are present, the carbohydrates of this structure are considered as component A when the weight-average chain length is below 6.5 and the individual members having a chain length of 7 and higher are not counted with the component A; on the other hand, they are considered as component B when the weight-average chain length is above 6.5 and then the individual members having a chain length of 6 and lower are not counted with the component B. Where both oligosaccharides and polysaccharides of the same structure are present in the absence of saccharides of another structure, there should be two maximums at either side of 7 units, or otherwise, the requirement of two different carbohydrate components is not met, as explained above.

The core of the mixtures may *inter alia* be seen in that carbohydrates of. With an administration of mixtures of carbohydrates of different sizes and/or different "classes" or "structures", a synergistic effect may occur relative to the prebiotic effects of the separate substance groups A and B.

The carbohydrates of component A may belong to one substance class alone but may also be formed out of several classes (for example A: galacto-oligosaccharides plus fuco-oligosaccharides), whereas the carbohydrates of component B may equally originate from one substance class and also from several substance classes (for example B: inulins plus xylans).

A preferred carbohydrate mixture is composed of galacto-oligosaccharide and inulin.

Particularly efficient mixtures are those wherein at least 60 wt%, preferably 80 to 100 wt% of carbohydrate components A belong to the group of galacto-oligosaccharides. Also preferred are mixtures wherein at least 60 wt%, preferably 80 to 100 wt% of the carbohydrate components B belong to the group of fructo-polysaccharides. For the production of the carbohydrate mixtures, carbohydrates and carbohydrate mixtures presently known and used in particular for the production of foods or food products can be used. It is also possible to use raw materials previously modified in a technical way. The preparation of the mixtures may thereby ensue by means of a simple blending of the correspondingly selected carbohydrates or oligosaccharides with polysaccharides or the carbohydrate mixtures. The initial components must thereby be so mixed with one another that the parameters are respected with the finished mixtures.

As raw materials may be used reserve carbohydrates (fructans, galacto-oligosaccharides from legumes, fucoidan, α-glucane, laminarin, carrageenan, mannans, galactomannans, agar), natural gum, N-glycosidic bonded carbohydrates of glycoproteins, O-glycosidic bonded carbohydrates of glycoproteins, glycans of glycolipids, enzymatically prepared carbohydrates (galacto-oligosaccharides, gluco-oligosaccharides, fructo-oligosaccharides, xylo-oligosaccharides), bacterial carbohydrates (such as xanthans), as well as oligosaccharides (galacto-oligosaccharides, gluco-oligosaccharides (from α 1-2 and α 1-3 glucose residues), xylo-oligosaccharides), as well as skeletal carbohydrates such as celluloses, hemicelluloses (arabinans, galactans), pectins and chitins may be used. The substances should preferably be of food-grade (cf. Complex Carbohydrates in Foods, British Nutrition Foundation; Chapman & Hall, London 1990).

It is also possible to carry out an enzymatic modification of the raw materials by means of hydrolases (e.g. glycosidases, transglycosidases and lipases), transferases, isomerases (e.g. aldolases and ketolases), oxidoreductases (e.g. oxidases) and reductases (e.g. glucosedehydrogenases), lyases (e.g. polysaccharide lyases) and ligases of the raw materials and products. Moreover, it is possible to carry out a technical modification of the raw materials and products, namely by means of pressure (e.g. extrusion), temperature (e.g. caramelisation), organic syntheses, organic modification (e.g. carboxymethylation and peracetylation), acid and/or alkaline hydrolysis and fractionation (e.g. depending on size and/or physico-chemical parameters such as charge and hydrophobicity) or combinations of modifications.

The carbohydrate mixtures thereby are essentially composed of the monosaccharides listed hereinafter and of the oligosaccharides and polysaccharides composed thereof: D-glucose, D-fructose, D-galactose, D-mannose, L-fucose, D-N-acetylglucosamine, D-N-acetylgalactosamine, D-xylose, L-rhamnose, D-arabinose, D-allose, D-talose, L-idose, D-ribose, as well as monosaccharides comprising carboxyl groups such as D-galacturonic acid, D-glucuronic acid, D-mannuronic acid and/or the methylated forms thereof such as N-acetylneuraminic acid, N-glycolylneuraminic acid and/or O-acetylated forms thereof. Moreover, these monomers and the higher units based thereon can be modified by means of -OSO₃H groups and/or -OPO₃H groups.

Non-digestible carbohydrates according to the present invention are typically administered at a daily dose of 0.5 to 30 g, preferably 2 to 15 g, more preferably 3 to 9 g.

One preferred mode of administration of the preparation is as a supplement. The supplement is suited for infants which are non-breast-fed or partly breast-fed, including non- or partly breast-fed prematurely born babies and non- or partly- breast-fed maturely born babies.

The preparation may also be used as an infant nutrition. In this case, the invention infant nutrition further comprises one or more ingredients selected from digestible carbohydrate, a lipid source, protein source, and mixtures thereof.

### 3) Other components

Apart from the carbohydrate components A and B, other carbohydrates may be present as well. Amongst those are 1) the digestible carbohydrates, which are digestible as described above, and 2) the insoluble carbohydrates, which are resorbable/digestible or even not resorbable/digestible. Typical insoluble non-digestible carbohydrates for use in the infant nutrition supplement are soy polysaccharides, and resistant starch, cellulose and hemicellulose; more preferably they are selected from soy polysaccharides and resistant starch.

Typical soluble and digestible carbohydrate for use in the infant nutrition supplement are selected from maltodextrins, starch, lactose, maltose, glucose, fructose, and sucrose and other mono- and disaccharides, and are more preferably selected from maltodextrin, lactose, maltose, glucose, fructose, sucrose, and mixtures thereof.

These carbohydrates enumerated *sub* 1) and 2), may be present as such in any arbitrary quantity in addition to the carbohydrate components A and B, in each case depending on the desired final product. Preferably, the insoluble carbohydrates constitute 0 to 10 wt% of the carbohydrate mixtures.

Typical ingredients for use as a lipid source for use in the infant nutrition supplement may be any lipid or fat which is suitable for use in infant formulas. Preferred lipid sources include milk fat, safflower oil, egg yolk lipid, canola oil, olive oil, coconut oil, palm oil, palm kernel oil, palm olein, soybean oil, sunflower oil, fish oil, and microbial fermentation oil containing long-chain polyunsaturated fatty acids. These oils may be in the form of high oleic form such as high oleic sunflower oil and high oleic safflower oil. The lipid source may also be in the form of fractions derived from these oils such as palm olein, medium chain triglycerides (MCT), and esters of fatty acids such as arachidonic acid, linoleic acid, palmitic acid, stearic acid, docosahexaeonic acid, linolenic acid, oleic acid, lauric acid, capric acid, caprylic acid, caproic acid, and the like.

For pre-term formulas, the lipid source preferably contains medium chain triglycerides, preferably in an amount of 15% to 35% by weight of the lipid source.

The lipid source preferably has a molar ratio of n-6 to n-3 fatty acids of 5:1 to 15:1, preferably from 8:1 to 10:1.

When present, it is preferred that the lipid are present at levels of from 20% to 40% by weight of the composition or as 0.8 to 1.5 g/100 kJ in an infant formula.

The proteins that may be utilised in the nutritional products of the invention include any protein or nitrogen source suitable for human consumption. Examples of suitable protein sources for use in infant formula typically include casein, whey, condensed skim milk, non-fat milk, soy, pea, rice, corn, hydrolysed protein, free amino acids, protein sources which contain calcium in a colloidal suspension with the protein and mixtures thereof. It is preferred for use herein that the protein are in hydrolysate form, thereby reducing the risk of allergy in such infant. Commercial protein sources are readily available and known to one practicing the art.

Typically, in the milk-based infant formula hydrolysates 100 % hydrolysed whey protein from cow's milk is present. In other milk-based infant formulae the ratio of casein/whey typically is between 1.8:0.3-3-0.

When present, it is preferred that the protein source is present at a levels of from 9% to 19% by weight of the composition. When used as an infant formula, the protein source is preferably present in an amount of from 0.45 to 1.0 g /100 kJ.

A nutritionally complete formula preferably contains all vitamins and minerals understood to be essential in the daily diet and in nutritionally significant amounts. Minimum requirements have been established for certain vitamins and minerals. Examples of minerals, vitamins and other nutrients optionally present in the infant formula include vitamin A, vitamin B, vitamin B2, vitamin B6, vitamin B12, vitamin E, vitamin K, vitamin C, vitamin D, folic acid, inositol, niacin, biotin, pantothenic acid, choline, calcium, phosphorous iodine, iron, magnesium, copper, zinc, manganese, chloride, potassium, sodium selenium, chromium, molybdenum, taurine, and L- carnitine. Minerals are usually added in salt form. The presence and amounts of specific minerals and other vitamins will vary depending on the intended infant population.

If necessary, the infant formula may contain emulsifiers and stabilisers such as soy lecithin, citric acid, esters of mono and di-glycerides, and the like. This is especially provided if the formula is to be provided in liquid form.

The infant formula may optionally contain other substances which may have a beneficial effect such as (non-carbohydrate) fibres, lactoferrin, immunoglobulins, nucleotides, nucleosides, and the like.

### Applications

The preparations according to the invention have been found to be particularly useful in the normalisation of the *Bifidobacterium* population according to the species distribution in breast-fed infants, considered as "standard", in the gastro-intestinal tract of infants which were non- or partly breast-fed, in particular those which are prematurely born babies, maturely born babies, as well as infants which are in the adaptation period to solid food. The preparation of the invention is also suitable for infants changing from breast to bottle feeding.

Accordingly, there is provided the use of the invention preparation or composition for the manufacture of a composition for the normalisation of the *Bifidobacterium* species population in the gastro-intestinal tract of non- or partly breast-fed infantsThe preparations of the invention have also been found particularly useful for the prevention or treatment of an immune condition. This immune condition is believed to be the result of the difference in the composition of the *Bifidobacterium* species in the gastrointestinal tract of these non- or partly breast-fed infants when compared to that of breast-fed infants.

Typically, such immune conditions include conditions selected from allergy, atopic dermatitis, eczema, asthma, atopy, allergic rhinitis, food hypersensitivity, diapers rashes, diarrhoea, and mixtures thereof.

Accordingly, the invention provides the use of the preparation for the prevention or treatment of one or more an immune conditions, preferably selected from allergy, atopic dermatitis, eczema, asthma, and diapers rashes,. Also (bacterial) diarrhoea and especially viral diarrhoea can be treated with the preparation of the invention. Also provided herein is the use of the preparation for the prevention and/or treatment of energy malabsorption.

Advantageously, the preparation has been found beneficial for inhibiting the infiltration of eosinophils, neutrophils and mononuclear cells in allergic lesions, and/or inhibiting the Th2 type immune response and/or stimulating the Th1 mediated immune response. Accordingly, there is provided the use of the invention preparation or composition as defined herein for the manufacture of a composition for inhibiting the infiltration of eosinophils, neutrophils and mononuclear cells in allergic lesions, inhibiting the Th2 type immune response and/or stimulating the Th1 mediated immune response.

The invention also provides the use of the carbohydrate mixture as described above for regulating the population of certain *Bifidobacterium* species other than *B. breve,* in particular for decreasing the relative amounts of *Bifidobacterium catenulatum, B. pseudocatenulatum* and/or *B. adolescentis.*

### Example 1: Validation of the developed probes and primers for bifidobacteria

The bacterial strains used to validate the assays for the relative quantification of the different *Bifidobacterium* species are listed in Table 2.

**Table 2**

| Bacterial strains and origins used for the development of the 5' nuclease assays | | | | |
|---|---|---|---|---|
| **Strain** | | **Origin^{a}** | | |
| *Bifidobacterium* strains | | | | |
| | *B. adolescentis* | ATCC 15703^{T} | ATCC 15705 | |
| | *B. angulatum* | DSM 20098^{T} | | |
| | *B. animalis* | ATCC 25527^{T} | DSM 10140 | |
| | *B. bifidum* | DSM 20456^{T} | NCIMB 8810 | |
| | *B. boum* | ATCC 27917^{T} | | |
| | *B. breve* | ATCC 15700^{T} | DSM 20091 | LMG 11613 |
| | *B. catenulatum* | ATCC 27539^{T} | ATCC 27675 | |
| | *B. dentium* | ATCC 27534^{T} | | |
| | *B. gallicum* | DSM 20093^{T} | | |
| | *B. gallinarum* | ATCC 33777^{T} | | |
| | *B. infantis* | LMG 8811^{T} | | |
| | *B. inopinatum* | DSM 10107^{T} | | |
| | *B. longum* | ATCC 15707^{T} | | |
| | *B. magnum* | ATCC 27540^{T} | | |
| | *B. pseudocatenulatum* | DSM 20438^{T} | | |
| | *B. pseudolongum* | ATCC 25526^{T} | | |
| | *B. suis* | ATCC 27533^{T} | | |

| Other Strains | | | | |
|---|---|---|---|---|
| | *Bacillus cereus* | ATCC 11778 | | |
| | *Bacteroides fragilus* | LMG 10263^{T} | | |
| | *Brevibacterium casei* | ATCC 35513^{T} | | |
| | *Clostridium difficile* | ATCC 9689^{T} | | |
| | *Enterococcus feacalis* | DSM 20478^{T} | | |
| | *Escherichia coli* | ATCC 35218 | | |
| | *Lactobacillus acidophilus* | ATCC 4356^{T} | | |
| | *Lactobacillus brevis* | LMG 18022 | | |
| | *Lactobacillus bulgaricus* | ATCC 11842^{T} | | |
| | *Lactobacillus casei* | ATCC 393^{T} | DSM 20011^{T} | |
| | *Lactobacillus fermentum* | DSM 20052^{T} | | |
| | *Lactobacillus plantarum* | DSM 20174^{T} | | |
| | *Lactobacillus reuteri* | LMG 9213^{T} | | |
| | *Lactobacillus rhamnosus* | ATCC 53103 | | |
| | *Listeria monocytogenes* | ATCC 7644 | | |
| | *Pediococcus acidilactici* | DSM 20284^{T} | | |
| | *Propionibacterium avidum* | DSM 4901 | | |
| | *Pseudomonas aeruginosa* | DSM 1117 | | |
| | *Saccharomyces cerevisiae* | DSM 2548 | | |
| | *Salmonella typhimurum* | ATCC 14028 | | |
| | *Staphylococcus aureus* | ATCC 29213 | | |

| | | | | |
|---|---|---|---|---|
| ATCC: American Type Culture Collection; DSM: Deutsche Sammlung von Mikroorganismen und Zellkulturen, Germany; LMG: Laboratory for Microbiology, University of Gent, Belgium; NCIMB: National Collections of Industrial and Marine Bacteria, UK. | | | | |

All bifidobacteria strains were cultured in Mann Rogosa Sharp (MRS) broth (Oxoid, Basingstoke, UK) media at 37°C for 24 hours under anaerobic conditions. The overnight cultures were stored at -20°C until further processing.

DNA was extracted from bacterial cultures by thawing 5 ml of frozen overnight cultures in ice water. Subsequently, the cultures were centrifuged for 20 minutes at 4000 rpm at 4 °C (Sorvall RT7, Du Pont, Stevenage, UK) to pellet the bacterial cells. The pellets were washed with 1 ml TES (50 mM Tris-HCl [pH 8.0], 5 mM EDTA, 50 mM NaCl), followed by a centrifugation step of 10 minutes at 4000 rpm at 4°C. Supernatants were removed and the pellet were resuspended in 1 ml of THMS (30 mM Tris-HCl [pH 8.0], 3 mM MgCl₂, 25% (w/v) sucrose). After transfer of the suspensions into a two ml eppendorf tube, 200 µl lysozyme (0.1 g/ml; Sigma Aldrich Chemie, Steinheim, DE) and 40 µl mutanolysine (1 mg/ml; Sigma Aldrich Chemie, DE) was added and incubated for 30 minutes at 37°C. Subsequently, the solutions were centrifuged for 5 minutes at 10000 rpm at 4 °C (Sigma 1-15, Sigma Laborzentrifugen GmbH, Osterode am Harz, DE). Supernatants were removed and the pellets were resuspended in 100 µl THMS, whereto 400 µl TES (including 0.5% SDS) and 7.5 µl of Proteïnase K (20 mg/ml; Boehringer Mannheim GmbH, Mannheim, DE) were added. The mixture was vortexed and incubated for 30 minutes at 65°C. Subsequently, a standard phenol/chloroform extraction was carried out, followed by a treatment with 2.5 µl RNase A (1 mg/ml; Roche Diagnostics, Mannheim, DE) for 30 minutes at 37°C. Subsequently, the DNA was precipitated by storing at -20°C for at least 30 minutes after addition of 2 volumes ice cold ethanol (96%) and 0.1 volume of 3 M sodium acetate (pH 5.2). Precipitated solutions were centrifuged for 20 minutes at 13000 rpm at 4 °C and the supernatants were washed with 500 µl 70% ethanol, followed by centrifugation at 13000rpm for 5 minutes at 4 °C.

Supernatants were discarded and the pellets were air dried at room temperature. The DNA was resuspended in 100 µl sterile milli-Q and stored at -20°C.

Firstly, the specificity of each duplex 5' nuclease assay was tested by performing a 25 µl amplification of the different strains (see table 2). These 25 µl PCR reactions were performed using 2.5 µl DNA template, 12.5 µl TaqMan Universal Master Mix (Applied Biosystems), 900 nM of each primer and 200 nM of each probe, followed by running the TaqMan Universal Temperature Profile, which consists of 2 minutes at 50 °C, 10 minutes at 95 °C, followed by 45 cycles of 15 seconds at 95 °C and 60°C for 1 minute, on the ABI Prism 7700 (Applied Biosystems, Nieuwerkerk a/d IJssel, NL). All of the 5' nuclease assays were specific for the *Bifidobacterium* species for which they were developed and the 5'nuclease assay for determination of the total amount of *Bifidobacterium* detected all *Bifidobacterium* species tested, but no other strains like *Propionibacterium* or *Lactobacillus*. It should be noted that the 5'nuclease assay for *B. catenulatum* also detects *B. pseudocatenulatum*. Furthermore, DNAse and RNAse treated samples were tested to assure that no contaminated RNA was detected during the assay.

Secondly, a mix of monocultures from *B. adolescentis, B. angulatum, B. breve, B. bifidum, B. catenulatum, B. dentium, B. infantis* and *B. longum* was prepared to verify that the total of this mix would sum up to approximately 100%. In that case, competition between the different *Bifidobacterium* species, which serve as template, can be excluded.

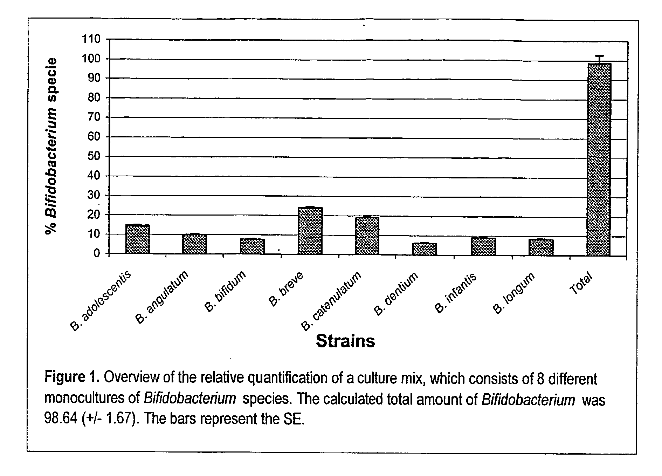

This is indeed the case, as can be seen in figure 1, which shows the determined amounts of each Bifidobacterium species in the mix as well as the total amount of *Bifidobacterium* species in the mix.

The CV values for reproducibility and repeatability for the different kind of 5' nuclease assays were determined and can be found in table 3.

**Table 3**

| Sensitivity of the 5' nuclease assays in comparison to "conventional" PCR and reproducibility and repeatability of the 5' nuclease assays | | | |
|---|---|---|---|
| Target | Sensitivity^{a} (x) | Reproducibility^{b} [CV (%)] | Repeatability^{c} [CV (%)] |
| *B. adolescentis* | 10,000 | 5.11 | 5.68 |
| *B. angulatum* | 1000 | 19.48 | 20.92 |
| *B. bifidum* | 100 | 11.65 | 11.20 |
| *B. breve* | 100 | 2.06 | 4.08 |
| *B. catenulatum* | 1000 | 9.42 | 14.83 |
| *B. dentium* | 100 | 12.65 | 11.35 |
| *B. infantis* | 1000 | 2.34 | 2.31 |
| *B. longum* | 10,000 | 9.10 | 8.18 |

| | | | |
|---|---|---|---|
| ^{a} number of times that the 5' nuclease assay is more sensitive then "conventional" PCR ^{b} reproducibility is determined by testing monocultures (100%) in ten fold and calculation of the CV (%) based on the gained results ^{c} repeatability is determined by testing monocultures (100%) three times in four fold and calculation of the CV (%) based on results gained | | | |

The developed 5' nuclease assays were compared to the conventional qualitative species-specific PCR (using the primers as described by Matsuki, T., K. Watanabe, R. Tanaka, M. Fukuda, and H. Oyaizu. 1999. Distribution of bifidobacterial species in human intestinal microflora examined with 16S rRNA-gene-targeted species-specific primers. Appl. Environ. Microbiol. 65:4506-4512) to determine the sensitivity of the different assays as well as checking for false positive or negative results. Table 3 shows the different sensitivities of the 5' nuclease assays in relation to the conventional species specific PCR. Table 4 show the final optimal primer and probe concentrations used in the duplex 5' nuclease assays.

**Table 4**

| Optimised final primer and probe concentrations used in the different duplex 5' nuclease assays | | | | |
|---|---|---|---|---|
| **Target** | **5' nuclease assay** | **Forward Primer (nM)** | **Reverse Primer (nM)** | **Probe (nM)** |
| *B. adolescentis* | *B. adolescentis* | 300 | 150 | 100 |
| | All *Bifidobacterium* | 300 | 600 | 100 |
| *B. angulatum* | *B. angulatum* | 900 | 900 | 200 |
| | All *Bifidobacterium* | 300 | 300 | 50 |
| *B. bifidum* | *B. bifidum* | 600 | 600 | 200 |
| | All *Bifidobacterium* | 300 | 300 | 100 |
| *B. breve* | *B. breve* | 300 | 300 | 100 |
| | All *Bifidobacterium* | 450 | 450 | 150 |
| *B. catenulatum* | *B. catenulatum* | 300 | 300 | 100 |
| | All *Bifidobacterium* | 600 | 600 | 100 |
| *B. dentium* | *B. dentium* | 900 | 900 | 200 |
| | All *Bifidobacterium* | 300 | 300 | 50 |
| *B. infantis* | *B. infantis* | 300 | 300 | 100 |
| | All *Bifidobacterium* | 900 | 900 | 100 |
| *B. longum* | *B. longum* | 300 | 300 | 100 |
| | All *Bifidobacterium* | 600 | 600 | 200 |
| All | All *Bifidobacterium* | 450 | 450 | 100 |
| *Bifidobacterium* | All bacteria | 900 | 900 | 200 |

### Example 2: Clinical trial

The study was a double blind, placebo-controlled multi-center trial with two intervention groups. Fully formula fed infants, aged 28 to 90 days, were recruited from four hospitals in Germany. Infants were included in the study if they had a birth weight between 2600 and 4500 g, and were fully formula fed for at least four weeks before the start of the intervention period. Infants with congenital abnormalities, or with proven or suspected cow's milk allergy, infants derived from multiple births, infants that had received antibiotics less than two weeks before the start of the study, and infants that were fed any pro- or prebiotic formula less than a month before the start of the study, were excluded from the study. After enrolment, infants were randomly allocated to one of two treatment groups: a group receiving an infant formula supplemented with 0,8 g/100ml galacto-oligosaccharides and fructo-polysaccharides (GFSF-group) and a group receiving a standard infant formula (SF-group). The macronutrient composition of the formulas is shown in table 5.

**Table 5:**

| Macronutrient composition of the study formulas (per 100 ml ready to use formula) | | |
|---|---|---|
| | Carbohydrate mixture-supplemented formula (Aptamil 1 with GOS/FOS, Milupa) | Standard formula (Aptamil 1, Milupa) |
| Energy (kcal) | 72 | 72 |
| Protein (g) | 1.5 | 1.5 |
| Carbohydrate (g) | 8.5 | 8.5 |
| -Lactose (g) | 7.5 | 7.5 |
| -Starch (g) | 1 | 1 |
| Non-digestible oligosaccharides (g) | 0.8 | 0 |
| -Galacto-oligosaccharides (g) | 0.72 | 0 |
| -Fructo-polysaccharides (g) | 0.08 | 0 |
| Fat (g) | 3.6 | 3.6 |

A group of breast-fed infants was included as a reference group (BF group). Within three days after the start of the study period, after 4 weeks, and at the end of the study period (6 weeks), faecal samples were collected. The study was approved by the medical ethical committees of the four hospitals. Written informed consent was obtained from the parents before the start of the study.

Nucleic acids were isolated from faeces by thawing faecal samples in ice water, followed by a 10x (w/v) dilution in PBS (0.37 M NaCl, 2.7 mM KCl, 8.1 mM Na₂HPO₄ [pH 7.4]) and homogenisation for 10 minutes using a stomacher (IUL Instruments, Barcelona, Spain). Homogenised faeces was stored at -20°C prior to the actual DNA isolation. The extractions were started by thawing 1 ml of a homogenised faeces sample in ice water, followed by centrifugation for 1 minute at 1100 rpm to remove debris and large particles. Supernatants were transferred to a new tube and centrifuged for 5 minutes at 10000 rpm. Subsequently, the pellets were resuspended in 1 ml TN150 (10 mM Tris-HCl [pH 8.0], 10 mM EDTA) and transferred to sterile tubes containing 0.3 g zirconium beads (diameter 0.1 mm, BioSpec Products, Bartlesville, US). To these suspensions 150 µl of TE-buffered phenol (pH ± 7.5) was added and the samples were placed in a mini-bead beater (BioSpec Products, Bartlesville, US), for 3 minutes at 5000 rpm. After bead-beating the samples were immediately cooled on ice, before addition of 150 µl chloroform. Samples were vortexed shortly and centrifuged for 5 minutes at 10000 rpm, upper phases were transferred to clean 2 ml eppendorf tubes and the phenol/chloroform extraction was started. Phenol-chloroform extraction was followed by precipitation of DNA through placement of the samples at -20 °C for at least 30 minutes, after addition of 1 ml ice-cold ethanol (96%) and 50 µl 3 M sodium acetate (pH 5.2). Consecutively, the samples were centrifuged for 20 minutes at 13000 rpm and washed with 500 µl 70% ethanol. After centrifugation for 5 minutes at 13000 rpm, the supernatants were discarded and the pellets were air dried at room temperature. The DNA was resuspended in 100 µl sterile milli-Q and stored at -20 °C.

The duplex 5' nuclease assays are used for the relative quantification of the different *Bifidobacterium* species in faecal samples. The relative amount of each species is calculated according to Liu et. al. 2002. Briefly, efficiency of each amplification curve was calculated separately, by the formula E = (threshold _{A} / threshold _{B})^{-(Ct,A - Ct,B)} - 1. With help of the calculated efficiencies the initial amount of DNA (Ro) is calculated by R₀ = threshold / (1 + E)^{Ct}. The initial amount of DNA of a *Bifidobacterium* species can then be divided with the initial amount of DNA of all *Bifidobacterium* species. Thereafter the obtained ratio's can be normalised with help of the ratio of a monoculture, which is set to 100%.

The total amount of *Bifidobacterium* was also determined with help of FISH, like earlier described (Langendijk, F. Schut, G. J. Jansen, G. C. Raangs, G. R. Kamphuis, M. H. Wilkinson and G. W. Welling "Quantitative fluorescence in situ hybridisation of Bifidobacterium spp. with genus-specific 16S rRNA-targeted probes and its application in fecal samples" Appl. Environ. Microbiol. 61(8):3069-75. (1995))

The percentage of the genus *Bifidobacterium* as a percentage of total bacteria was 75, 47, and 68% in the BF, SF, and GFSF group, respectively, which demonstrates that the GFSF group, fed a mixture of nondigestible carbohydrates, has a more bifidogenic flora, as in the BF group, than in the SF group.

In table 6 the prevalence of each species in the different groups at the beginning as well as at the end of the study is shown. In table 7 the percentage of *Bifidobacteria* species relative to the total amount of *Bifidobacteria* is shown.

**Table 6:**

| Prevalence (in %) of *Bifidobacteria* species in the faeces of infants after 6 weeks of feeding with human milk (BF), an infant formula with a prebiotic mixture (GFSF) or with a standard formula (SF). | | | |
|---|---|---|---|
| Species | BF | GFSF | SF |
| *B. catenulatum* | 80 | 67 | 75 |
| *B. adolescentis* | 20 | 11 | 50 |
| *B. breve* | 70 | 78 | 63 |
| *B. longum* | 50 | 56 | 63 |
| *B. bifidum* | 10 | 11 | 13 |
| *B. angulatum* | 30 | 11 | 13 |
| *B. infantis* | 100 | 100 | 100 |
| *B. dentium* | 20 | 11 | 13 |

**Table 7:**

| Percentage of *Bifidobacteria* species with respect to the total number of *Bifidobacteria* in the faeces after a 6 week feeding period. | | | |
|---|---|---|---|
| | Breast-fed | GFSF-fed | SF-fed |
| Species | % (sd) | % (sd) | % (sd) |
| *B. catenulatum* | 1.9 (1.0) | 1.5 (3.0) | 9.8 (12.6) |
| *B. adolescentis* | 0.3 (0.9) | 0.1 (0.2) | 2.9 (6.0) |
| *B. breve* | 11.7 (9.6) | 5.4 (10.8) | 4.9 (10.7) |
| *B. longum* | 7.3 (13.9) | 5.4 (10.7) | 6.2 (9.4) |
| *B. bifidum* | <0.1 (0.0) | <0.1 (0.0) | <0.1 (0.0) |
| *B. angulatum* | <0.0 (0.0) | <0.1 (0.2) | <0.1 (0.0) |
| *B. infantis* | 32.0 (18.9) | 32.1 (20.0) | 37.8 (18.4) |
| *B. dentium* | <0.1 (0.0) | <0.1 (0.0) | <0.1 (0.0) |

A large variety of *Bifidobacterium* species is present in the three different groups. Furthermore, a significant decrease in prevalence and amount of *B. adolescentis* is visible in breast-fed infants and in infants receiving GFSF contrary to infants receiving a standard formula. After 6 weeks of feeding the prevalence and percentage of *B. adolescentis* is much higher in SF-fed babies than in babies which were GFSF or breast-fed. Analyses of the faecal samples of GFSF infants shows a large variety in the bifidobacterial flora similar to breast-fed infants and stimulation of only one or a few species is not observed. Besides the effect on *B. adolescentis* the profiles of breast-fed infants and infants receiving GFSF also showed less *B. catenulatum* (+ *B. pseudocatenulatum)* than the profile of infants receiving a standard formula. *B. infantis*, and *B. longum* seems to be predominant in breast-fed infants as well as in infants receiving a standard formula (SF) or a standard formula supplemented with prebiotics (GFSF). Also *B. breve* was dominant in all three groups, but in the group receiving breast milk *B. breve* as a % of total bifidobacteria was higher (11.7 %) as in the SF (4.9 % ) and GFSF (5.4%) group.

### Example 3: Animal experiments on allergy

Specific pathogen free male BALB/c mice were obtained from Charles River (Maastricht, the Netherlands). Food and water was provided *ad libitum* and the mice were used when 6-9 weeks of age. All experiments were approved by the animal ethics committee of the University of Utrecht, The Netherlands.

Ovalbumin (grade V) and acetyl-β-methylcholine chloride (methacholine) were purchased from Sigma Chemical Co. (St. Louis, MO, USA). Aluminium hydroxide (AlumImject) was purchased from Pierce (Rockford, IL, USA).

Mice were sensitised by two i.p. injections with 10 µg ovalbumin adsorbed onto 2.25 mg aluminium hydroxide in 100 µl saline or saline alone on days 0 and 7. Mice were challenged on days 35, 38, and 41 by inhalation of ovalbumin aerosols in a plexiglass exposure chamber for 20 minutes. The aerosols were generated by nebulising an ovalbumin solution (10 mg/ml) in saline using a Pari LC Star nebulizer (Pari respiratory Equipment, Richmond, VA, USA).

Mice were treated daily with 1x10e9 (CFU) *Bifidobacterium breve* and 25 mg of a mixture of galactooligosaccharides and fructopolysaccharides (9:1) orally via gavage (0.2 ml, physiological salt solution) starting at day 28 upto the end of the experiment (i.e. day 42). As a control 0.2 ml physiological salt solution was administered via gavage.

Airway responsiveness to inhaled nebulised methacholine was determined 24 hours after the final aerosol challenge, in conscious, unrestrained mice using whole body plethysmo-graphy (BUXCO, EMKA, Paris, France). The airway response was expressed as enhanced pause (PenH).

Statistical Analysis: The airway response curves to methacholine were statistically analysed by a general linear model or repeated measurements followed by post-hoc comparison between groups. Cell counts were statistically analysed using the Mann-Whitney U test (Siegel, S., Castellan Jr. N J, 1988, "Nonparametric statistics for the behavioural sciences" 2nd ed. McGraw Hill Book Company, New York, USA). All other analyses were performed using Student's t-test (Abramowitz, M., Stegun, I.A., 1972, "Handbook of mathematical functions" Dover publications, Inc. New York, USA). A probability value of p<0.05 was considered as statistically significant.

Results on airway hyperresponsiveness: Measurements on airway hyperresponsiveness show that compared to control the mice receiving the *B. breve* + a mixture of galactooligosaccharides and fructopolysaccharides show a statistically reduced airway hyperresponsiveness, indicative of a lowered asthmatic reaction.

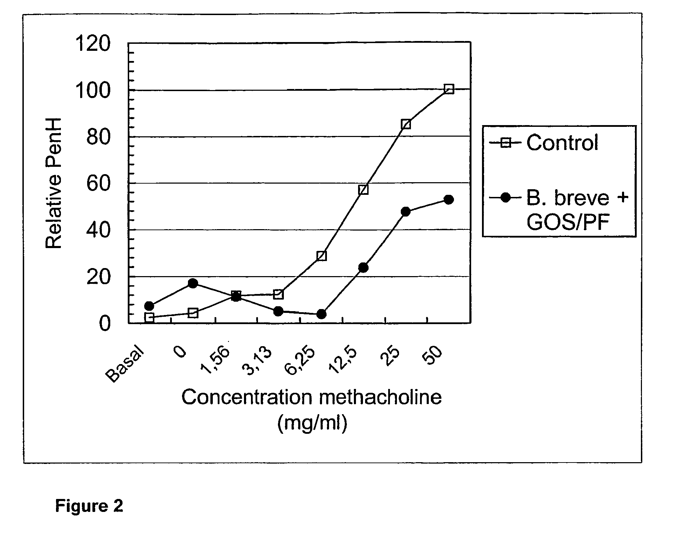

In figure 2 the airway hyperresponsiveness is plotted as relative PenH (enhanced pause) versus the metacholine concentration for mice receiving a combination of *B. Breve* + a mixture of GOS/FOS and a control group of mice receiving saline instead. The plotted values of relative PenH are obtained after subtraction of the blank values obtained for mice not ovalbumin-sensitised and normalisation to the value obtained for the control group at the highest concentration of metacholine.

The compositions of all following examples may additionally contain minerals, trace elements and vitamins, choline, taurine, carnitine, and/or myo-inositol or mixtures thereof, as known in the art and in accordance with international guidelines. Furthermore, organic acids, flavours and or colorants may or may not be present.

### Example 4

An infant milk formula containing per 100 ml final product (and per 13.1 g powder):

| | |
|---|---|
| 8 energy % protein | 1.4 g (casein whey mixture) |
| 45 energy % digestible carbohydrates | 7.5 g |
| 47 energy % fat | 3.5 g |
| GOS (90% galacto-oligosaccharides, Borculo Domo NL) / polyfructose, (10% inulin, Raftilin HP, Orafti BE) | 0.4 g |
| *B. breve:* | 1.3x10⁸ cfu |

### Example 5

An infant milk formula containing per 100 ml final product (and per 14 g powder):

| | |
|---|---|
| 10 energy % protein | 1.8 g (casein whey mixture) |
| 46 energy % digestible carbohydrates | 8.0 g |
| 44 energy % fat | 3.4 g |
| GOS/polyfructose (see ex. 4) | 0.4 g |
| *B. breve* | 1.4x10⁸ cfu |

### Example 6

An infant milk formula containing per 100 ml final product (and per 16.1 g powder):

| | |
|---|---|
| 10 energy % protein | 1.9 g (casein whey mixture) |
| 51 energy % digestible carbohydrates | 9.9 g |
| 39 energy % fat | 3.3 g |
| FOS (Raftilin, Orafti) / galactomannan 9/1 | 0.4 g |
| *B. breve* | 1.6x10⁸ cfu |

### Example 7

An infant milk formula containing per 100 ml final product (and per 13 g powder):

| | |
|---|---|
| 10 energy % protein equivalent | 1.6 g (hydrolysed whey protein) |
| 42 energy % digestible carbohydrates | 7.1 g |
| 48 energy % fat | 3.6 g |
| sialyl oligosaccharides, indigestible maltodextrins 9/1 | 0.4 g |
| *B. breve* | 6.5x10⁸ cfu/g |

### Example 8

An infant milk formula containing per 100 ml fmal product (and per 15 g powder):

| | |
|---|---|
| 10 energy % protein equivalent | 1.8 g (hydrolysed whey protein) |
| 42 energy % digestible carbohydrates | 8.6 g |
| 44 energy % fat | 3.6 g |
| fuco-oligosaccharides(from algal fucoidan), galactomannan 8/2 | 0.4 g |
| *B. breve* | 7.5x10⁸ cfu |

### Example 9

An infant milk formula containing per 100 ml final product (and per 15.1 g powder):

| | |
|---|---|
| 10 energy % protein equivalent | 1.8 g (hydrolysed whey protein) |
| 42 energy % digestible carbohydrates | 8.6 g |
| 44 energy % fat | 3.6 g |
| manno-oligosaccharides, arabinogalactan 9/1 | 0.4 g |
| *B. breve* | 1.5x10⁸ cfu |

### Example 10

An infant milk formula containing per 100 ml final product (and per 15.2 g powder):

| | |
|---|---|
| 10 energy % protein | 1.7 g (hydrolysed whey protein) |
| 48 energy % digestible carbohydrates | 8.4 g |
| 42 energy % fat | 3.3 g |
| GOS/galactoruonic oligo-saccharides//polyfructose 7/2/1 | 1.0 g |
| *B. breve* | 7.5x10⁸ cfu |

### Example 11

An infant milk formula containing per 100 ml final product (and per 15.8 g powder):

| | |
|---|---|
| 11 energy % protein | 1.9 g (hydrolysed whey protein) |
| 48 energy % digestible carbohydrates | 8.7 g |
| 41 energy % fat | 3.3 g |
| xylooligosaccharides/galactan 9/1 | 0.8 g |
| *B. breve* | 8x10⁸ cfu |

### Example 12

An infant milk formula containing per 100 ml final product (and per 15 g powder):

| | |
|---|---|
| 10 energy % protein | 1.7 g (casein whey mixture) |
| 48 energy % digestible carbohydrates | 8.1 g |
| 42 energy % fat | 3.1 g |
| GOS/polyfructose 9/1 | 0.8 g |
| Galactomannan | 0.42 |
| *B. breve* | 1.5x10⁸ cfu |

### Example 13

An infant milk formula containing per 100 ml final product (and per 15.9 g powder):

| | |
|---|---|
| 13 energy % protein | 2.2 g (casein whey mixture) |
| 49 energy % digestible carbohydrates | 8.6 g |
| 37 energy % fat | 3.0 g |
| GOS/polyfructose 9/1 | 0.8 g |
| Galactomannan | 0.4 g |
| *B. breve* | 1.6x10⁸ cfu |

### Example 14

An infant milk formula containing per 100 ml final product (and per 13.5 g powder):

| | |
|---|---|
| 9 energy % protein equivalent | 1.5 g (hydrolysed whey protein) |
| 42 energy % digestible carbohydrates | 6.9 g |
| 49 energy % fat | 3.6 g |
| GOS/polyfructose/sialyllactose 7/2/1 | 0.8 g |
| *B. breve* | 1.4x10⁸ cfu |

### Example 15

An infant milk formula containing per 100 ml final product (and per 13.7 g powder):

| | |
|---|---|
| 9 energy % protein equivalent | 1.4 g (free amino acids) |
| 44 energy % digestible carbohydrates | 7.1 g |
| 47 energy % fat | 3.4 g |
| GOS/polyfructose 6/4 | 0.8 g |
| *B. breve* | 1.4x10⁸ cfu |

### Example 16

An infant formula containing per 100 ml final product (and per 13.5 g powder):

| | |
|---|---|
| 11 energy % protein | 1.8 g (soy protein) |
| 40 energy % digestible carbohydrates | 6.7 g |
| 49 energy % fat | 3.6 g |
| GOS/galacto-ogosaccharides/polyfructose 8/1/1 | 0.8 g |
| *B. breve* | 1.4x10⁸ cfu |

### Example 17

An infant formula containing per 100 ml final product (and per 15.1 g powder):

| | |
|---|---|
| 12 energy % protein | 2.2 g (soy protein) |
| 43 energy % digestible carbohydrates | 7.7 g |
| 45 energy % fat | 3.6 g |
| FOS/galactan 9/1 | 0.8 g |
| *B. breve* | 1.5x10⁸ cfu |

### Example 18

An infant formula containing per 100 ml (and 16.5 g powder)

| | |
|---|---|
| 13 energy % protein | 2.0 g (hydrolysed whey) |
| 57 energy % digestible carbohydrates | 8.6 g |
| 30 energy % fat | 2.0 g |
| GOS/polyfructose 9/1 | 1.0 |
| Soy polysaccharides | 0.5 |
| *B. breve* | 1.5x10⁹ cfu |

### Example 19

A milk-based product containing per 100 ml

| | |
|---|---|
| 14 energy % protein | 2.5 g (cow's milk protein) |
| 43 energy % digestible carbohydrates | 7.5 g |
| 43 energy % fat | 3.4 g |
| GOS/polyfructose 7/3 | 1.5 g |
| *B. breve* | 3x10⁸ cfu |

### Example 20

An infant formula containing per 100 ml (and 15.4 g powder)

| | |
|---|---|
| 11 energy % protein | 2.0 g (hydrolysed collagen and soy protein) |
| 46 energy % digestible carbohydrates | 8.6 g |
| 43 energy % fat | 3.6 g |
| GOS/polyfructose 3/1 | 0.4 g |
| *B_{.} breve* | 6x10⁸ cfu |

### Example 21

A supplement: 3 g powder to be added to 100 ml milk: containing:

| | |
|---|---|
| 28 energy % protein | 0.7 g (casein whey mixture) |
| 72 energy % digestible carbohydrates | 2.0 g |
| GOS/polyfructose 65/35 | 0.3 g |
| *B. breve* | 3x10⁹ cfu |

### Example 22

A supplement containing: 0.4-0.8 g to be added to 100 ml milk: per g:

| | |
|---|---|
| 0.26 g | galactomannan, |
| 0.44 g | digestible carbohydrates |
| 0.3 g | GOS/polyfructose 85/15 |
| 1.0x10⁹ cfu | *B. breve* |

### Example 23

A supplement containing per 100 ml

| | |
|---|---|
| 100 % energy digestible carbohydrates | 2.2 g |
| minerals: | K, Na, Cl, |
| osmolarity | 261 mOsm/l |
| GOS/polyfructose 55/45 | 0.4 g |
| *B. breve* | 1x10⁹ cfu |

### Example 24

An infant nutrition containing per 100 g (85 g to be added to 240 ml milk)

| | |
|---|---|
| 4 energy % protein | 4.7 g (cow's milk protein) |
| 53 energy % digestible carbohydrates | 68 g |
| 43 energy % fat | 24.6 g |
| GOS/polyfructose 9/1 | 0.8 g |
| *B. breve* | 1.2x10⁹ cfu |

### Example 25

An infant nutrition (tube feed): per 100 ml

| | |
|---|---|
| 9 energy % protein | 3.4 g (casein) |
| 50 energy % carbohydrates | 18.8 g |
| 41 energy % fat | 8 g |
| GOS/polyfructose 7/3 | 0.4 g |
| *B. breve* | 5x10⁸ cfu |

### Example 26

An infant nutrition containing per 100 ml product

| | |
|---|---|
| 11 energy % protein | 2.8 g (casein) |
| 49 energy % carbohydrates | 12.3 g |
| 40 energy % fat | 4.4 g |
| GOS/polyfructose 85/15 | 0.8 g |
| *B. breve* | 5x10⁸ cfu |

### Example 27

An infant nutrition composed of rice flour containing per 100 g dry product: (4-7 spoons to be added to 200 ml warm infant formula, follow-on formula, toddler's milk or cow's milk)

| | |
|---|---|
| 7.4 g | protein (vegetable) |
| 83 g | carbohydrates |
| 0.5 g | fat |
| 3 g | dietary fibre including 1.5 g GOS/polyfructose 9/1 |
| 1x10¹⁰ cfu *B. breve* | |

### Example 28

An infant nutrition composed of precooked flakes (wheat, rye, rice, barley, maize, oat, buckwheat) containing per 100 g dry product. (5-7 spoons to be added to 250 ml warm infant formula, follow-on formula, toddler's milk or cow's milk)

| | |
|---|---|
| 9.5 g | protein (vegetable) |
| 74 g | carbohydrates |
| 2.0 g | fat |
| 3 g | dietary fibre including 1.5 g GOS/polyfructose 8/2 |
| 2x10¹⁰ cfu *B. breve* | |

### Example 29

An infant nutrition composed of homogenised vegetables or fruit, containing per 100 ml

| | |
|---|---|
| GOS/polyfructose 75/25 | 2.0 g |
| *B. breve* | 2x10⁹ cfu |

## Claims

1. A preparation comprising *Bifidobacterium breve* and a mixture of at least two non-digestible soluble carbohydrate components A and B, wherein
- said carbohydrate component A has a different structure of the monosaccharide units of the carbohydrate from said carbohydrate component B;
- said carbohydrate component A is present in an amount of 5 to 95 % by weight of the sum of carbohydrate components A and B;
- at least 50% of the total non-digestible soluble carbohydrates being selected from disaccharides to eicosasaccharides; and
- carbohydrate components A and B differ in the number of monosaccharide units, component A having an average chain length which is at least 5 monosaccharide units lower than the average chain length of component B.

2. A preparation according to claim 1, wherein said carbohydrate component A is selected from indigestible monosaccharides up to hexasaccharides of the same carbohydrate structure, and said component B is selected from indigestible heptasaccharides and higher polysaccharides of the same carbohydrate structure.

3. A preparation according to any one of claims 1-2, wherein the carbohydrate component A comprises 95 to 60 wt% and the carbohydrate B comprises 5 to 40 wt%, with A + B = 100 wt%.

4. A preparation according to any one of claims 1-3, wherein at least 60 wt%, preferably 80 to 100 wt% of said carbohydrate component A belong to the group of galacto-oligosaccharides, preferably to the group of trans-galacto-oligosaccharides.

5. A preparation according to any one of claims 1-4, wherein at least 60 wt%, preferably 80 to 100 wt% of said carbohydrate component B belong to the group of fructo-polysaccharides, including inulin.

6. A preparation according to any one of claims 1-5, comprising 10⁵ to 10¹¹ cfu of *Bifidobacterium breve* per g of total non-digestible soluble carbohydrate.

7. A preparation according to any one of claims 1-6 for use as a supplement, wherein the probiotic *Bifidobacterium breve* is present in the supplement in an amount of 1x10⁵ to 1.5x10¹¹ cfu/g, calculated on the basis of the supplement.

8. A preparation according to any one of claims 1-6 for use as an infant nutrition, wherein the *Bifidobacterium breve* is present in the supplement in an amount of 1x10² to 1x10¹² cfu/g of the infant supplement.

9. An infant nutrition supplement comprising a preparation according to any one of claims 1-7, and further comprising digestible carbohydrate, a lipid source, or a protein source, or a mixture thereof.

10. An infant nutrition comprising a preparation according to any one of claims 1-6 and 8, and further comprising digestible carbohydrate, a lipid source, and a protein source.

11. Use of a preparation according to any one of claims 1-8 for the manufacture of a composition for the normalisation of the *Bifdobacterium* species composition in the gastro-intestinal tract of non- or partially breast-fed infants to the composition in breast-fed infants.

12. Use of a preparation according to any one of claims 1-8 for the manufacture of a composition for the prevention or treatment of one or more immune disorders.

13. Use according to claim 12, wherein said immune disorders are selected from allergy, atopy, allergic rhinitis, food hypersensitivity, atopic dermatitis, eczema and asthma.

14. Use according to claim 12 or 13, wherein said immune disorders are selected from diarrhoea and viral diarrhoea.

15. Use of a preparation according to any one of claims 1-8 for the manufacture of a composition for preventing and/or treating energy malabsorption.

16. Use of a preparation according to any one of claims 1-8 for the manufacture of a composition for inhibiting the infiltration of eosinophils, neutrophils and mononuclear cells in allergic lesions, inhibiting the Th2 type immune response and/or stimulating the Th1 mediated immune response.

17. Use of a mixture of at least two non-digestible soluble carbohydrate components A and B, wherein
- said carbohydrate component A is present in an amount of 5 to 95 % by weight of the sum of carbohydrate components A and B;
- at least 50% of the total non-digestible soluble carbohydrates is selected from disaccharides to eicosasaccharides; and
- said carbohydrate components A and B differ (i) in the average number of monosaccharide units of the carbohydrate, (ii) and said carbohydrate component A has a different structure of the monosaccharide units of the carbohydrate from said carbohydrate component B
for the manufacture of a composition for decreasing the relative amounts of *Bifidobacterium catenulatum, B. pseudocatenulatum* and/or *B. adolescentis* with respect to the total number of *Bifidobacteria* in the gastro-intestinal tract of non- or partially breast-fed infants.

## Patentansprüche

1. Zubereitung umfassend *Bifidobacterium breve* und eine Mischung von mindestens zwei nicht verdaulichen löslichen Kohlenhydratkomponenten A und B, wobei
- die Kohlenhydratkomponente A eine andere Struktur der Monosaccharideinheiten des Kohlenhydrats als die Kohlenhydratkomponente B aufweist;
- die Kohlenhydratkomponente A in einer Menge von 5 bis 95 Gew.-%, auf die Summe der Kohlenhydratkomponenten A und B bezogen, vorliegt;
- mindestens 50 % der gesamten nicht verdaulichen löslichen Kohlenhydrate unter Disacchariden bis Eicosacchariden ausgewählt sind; und
- die Kohlenhydratkomponenten A und B bezüglich der Anzahl von Monosaccharideinheiten verschieden sind, wobei die Komponente A eine durchschnittliche Kettenlänge aufweist, die mindestens 5 Monosaccharideinheiten kürzer ist als die durchschnittliche Kettenlänge der Komponente B.

2. Zubereitung nach Anspruch 1, wobei die Kohlenhydratkomponente A unter unverdaulichen Monosacchariden bis zu Hexasacchariden derselben Kohlenhydratstruktur ausgewählt wird und die Komponente B unter unverdaulichen Heptasacchariden und höheren Polysacchariden derselben Kohlenhydratstruktur ausgewählt wird.

3. Zubereitung nach einem der Ansprüche 1 - 2, wobei die Kohlenhydratkomponente A 95 bis 60 Gew.-% ausmacht und das Kohlenhydrat B 5 bis 40 Gew.-% ausmacht, wobei A + B = 100 Gew.-% betragen.

4. Zubereitung nach einem der Ansprüche 1 - 3, wobei mindestens 60 Gew.-%, bevorzugt 80 bis 100 Gew.-% der Kohlenhydratkomponente A zur Gruppe der Galactooligosaccharide, bevorzugt zur Gruppe der Transgalactooligosaccharide gehören.

5. Zubereitung nach einem der Ansprüche 1 - 4, wobei mindestens 60 Gew.-%, bevorzugt 80 bis 100 Gew.-% der Kohlenhydratkomponente B zur Gruppe der Fructopolysaccharide, einschließlich Inulin, gehören.

6. Zubereitung nach einem der Ansprüche 1 - 5, umfassend 10⁵ bis 10¹¹ KbE *Bifidobacterium breve* pro g nicht verdaulichem löslichem Kohlenhydrat insgesamt.

7. Zubereitung nach einem der Ansprüche 1 - 6 zur Verwendung als Nahrungsmittelergänzung, wobei das probiotische *Bifidobacterium breve* in der Nahrungsmittelergänzung in einer Menge von 1 x 10⁵ bis 1,5 x 10¹¹ KbE/g, auf der Basis der Nahrungsmittelergänzung berechnet, vorliegt.

8. Zubereitung nach einem der Ansprüche 1 - 6 zur Verwendung als Säuglingsernährung, wobei das probiotische *Bifidobacterium breve* in der Nahrungsmittelergänzung in einer Menge von 1 x 10² bis 1 x 10¹² KbE/g, auf die Säuglingsnahrungsmittelergänzung bezogen, vorliegt.

9. Säuglingsnahrungsmittelergänzung umfassend eine Zubereitung nach einem der Ansprüche 1 - 7, und des Weiteren umfassend verdauliches Kohlenhydrat, eine Lipidquelle oder eine Proteinquelle oder eine Mischung derselben.

10. Säuglingsernährung umfassend eine Zubereitung nach einem der Ansprüche 1 - 6 und 8, und des Weiteren umfassend verdauliches Kohlenhydrat, eine Lipidquelle und eine Proteinquelle.

11. Verwendung einer Zubereitung nach einem der Ansprüche 1 - 8 zur Herstellung einer Zusammensetzung zum Normalisieren der Zusammensetzung der *Bifidobakterium*-Spezies im Magen-Darmkanal von nicht- oder teilweise gestillten Säuglingen zur Zusammensetzung in gestillten Säuglingen.

12. Verwendung einer Zubereitung nach einem der Ansprüche 1 - 8 zur Herstellung einer Zusammensetzung zur Prävention oder Behandlung einer oder mehrerer Immunstörungen.

13. Verwendung nach Anspruch 12, wobei die Immunstörungen unter Allergie, Atopie, allergischer Rhinitis, Nahrungsmittelüberempfindlichkeit, atopischer Dermatitis, Ekzem und Asthma ausgewählt sind.

14. Verwendung nach Anspruch 12 oder 13, wobei die Immunstörungen unter Diarrhö und Virusdiarrhö ausgewählt sind.

15. Verwendung nach einem der Ansprüche 1 - 8 zur Herstellung einer Zusammensetzung zur Prävention und/oder Behandlung von Energiemalabsorption.

16. Verwendung einer Zubereitung nach einem der Ansprüche 1 - 8 zur Herstellung einer Zusammensetzung zum Hemmen der Infiltration von Eosinophilen, Neutrophilen und mononuklearen Zellen in allergischen Läsionen, Hemmen der Immunantwort vom Th2-Typ und/oder Stimulieren der durch Th1 vermittelten Immunantwort.

17. Verwendung einer Mischung von mindestens zwei nicht verdaulichen löslichen Kohlenhydratkomponenten A und B, wobei
- die Kohlenhydratkomponente A in einer Menge von 5 bis 95 Gew.-%, auf die Summe der Kohlenhydratkomponenten A und B bezogen, vorliegt;
- mindestens 50 % der gesamten nicht verdaulichen löslichen Kohlenhydrate unter Disacchariden bis Eicosacchariden ausgewählt sind; und
- die Kohlenhydratkomponenten A und B (i) bezüglich der durchschnittlichen Anzahl von Monosaccharideinheiten des Kohlenhydrats verschieden sind, (ii) und die Kohlenhydratkomponente A eine andere Struktur der Monosaccharideinheiten des Kohlenhydrats als die Kohlenhydratkomponente B aufweist,
für die Herstellung einer Zusammensetzung zum Reduzieren der relativen Mengen von *Bifidobacterium catenulatum, B. pseudocatenulatum* und/oder *B. adolescentis* mit Bezug auf die gesamte Anzahl von Bifidobakterien im Magen-Darmkanal von nicht- oder teilweise gestillten Säuglingen.

## Revendications

1. Préparation comprenant du *Bifidobacterium breve* et un mélange d'au moins deux composants de glucide solubles non-digestibles A et B, où
- ledit composant de glucide A présente une structure différente des unités de monosaccharide du glucide dudit composant de glucide B ;
- ledit composant de glucide A est présent en une quantité de 5 à 95% en poids de la somme des composants de glucide A et B ;
- au moins 50% des glucides solubles non-digestibles totaux étant sélectionnés parmi des disaccharides à des eicosasaccharides ; et
- les composants de glucide A et B diffèrent en nombre d'unités de monosaccharide, le composant A ayant une longueur de chaîne moyenne qui est au moins 5 unités de monosaccharide plus basse que la longueur de chaîne moyenne du composant B.

2. Préparation selon la revendication 1, où ledit composant de glucide A est sélectionné parmi des monosaccharides indigestibles jusqu'à des hexasaccharides de la même structure de glucide, et ledit composant B est sélectionné parmi des heptasaccharides indigestibles et des polysaccharides plus élevés de la même structure de glucide.

3. Préparation selon l'une quelconque des revendications 1-2, où le composant de glucide A comprend 95 à 60% en poids et le composant de glucide B comprend 5 à 40% en poids, A + B = 100% en poids.

4. Préparation selon l'une quelconque des revendications 1 à 3, où au moins 60% en poids, de préférence 80 à 100% en poids dudit composant de glucide A appartiennent au groupe de galacto-oligosaccharides, de préférence au groupe de trans-galacto-oligosaccharides.

5. Préparation selon l'une quelconque des revendications 1 à 4, où au moins 60% en poids, de préférence 80 à 100% en poids dudit composant de glucide B appartiennent au groupe de fructo-polysaccharides, incluant l'inuline.

6. Préparation selon l'une quelconque des revendications 1 à 5, comprenant 10⁵ à 10¹¹ cfu de *Bifidobacterium breve* par g de glucide soluble non digestible total.

7. Préparation selon l'une quelconque des revendications 1 à 6 pour utilisation comme un supplément, où le *Bifidobacterium breve* probiotique est présent dans le supplément en une quantité de 1x10⁵ à 1,5x10¹¹ cfu/g, calculé sur la base du supplément.

8. Préparation selon l'une quelconque des revendications 1 à 6 pour utilisation comme nutrition pour nourrisson, où le Bifidobacterium breve est présent dans le supplément en une quantité de 1x10² à 1x10¹² cfu/g du supplément pour nourrisson.

9. Supplément de nutrition pour nourrisson comprenant une préparation selon l'une quelconque des revendications 1 à 7, et comprenant en outre du glucide digestible, une source de lipide ou une source de protéine ou un mélange de ceux-ci.

10. Nutrition pour nourrisson comprenant une préparation selon l'une quelconque des revendications 1 à 6 et 8, et comprenant en outre du glucide digestible, une source de lipide et une source de protéine.

11. Utilisation d'une préparation selon l'une quelconque des revendications 1 à 8 pour la fabrication d'une composition pour la normalisation de la composition de l'espèce *Bifidobacterium* dans la voie gastro-intestinale de nourrissons non ou partiellement nourris au sein à la composition de nourrissons nourris au sein.

12. Utilisation d'une préparation selon l'une quelconque des revendications 1 à 8 pour la fabrication d'une composition pour empêcher ou traiter un ou plusieurs troubles d'immunité.

13. Utilisation selon la revendication 12, où lesdits troubles d'immunité sont sélectionnés parmi l'allergie, l'atopie, rhinite allergique, hypersensibilité aux aliments, dermatite atopique, eczéma et asthme.

14. Utilisation selon la revendication 12 ou 13, où lesdits troubles d'immunité sont sélectionnés entre la diarrhée et la diarrhée virale.

15. Utilisation d'une préparation selon l'une quelconque des revendications 1 à 8 pour la fabrication d'une composition pour empêcher et/ou traiter une malabsorption d'énergie.

16. Utilisation d'une préparation selon l'une quelconque des revendications 1 à 8 pour la fabrication d'une composition pour empêcher l'infiltration de cellules eosinophiles, neutrophiles et mononucléaires dans des lésions allergiques, empêchant la réponse d'immunité de type Th2 et/ou la stimulation de la réponse d'immunité par Th1.

17. Utilisation d'un mélange d'au moins deux glucides solubles non-digestibles A et B, où
- ledit composant de glucide A est présent en une quantité de 5 à 95% en poids de la somme des composants de glucide A et B ;
- au moins 50% des glucides solubles non-digestibles totaux est sélectionné parmi des disaccharides à des eicosasaccharides ; et
- lesdits composants de glucide A et B diffèrent (i) dans le nombre moyen d'unités de monosaccharides du glucide, (ii) ledit composant de glucide A a une différente structure des unités de monosaccharide du glucide dudit composant de glucide B
pour la fabrication d'une composition pour diminuer les quantités relatives de *Bifidobacterium catenulatum, B. pseudocatenulatum* et/ou *B. adolescentis* par rapport au nombre total de *Bifidobacteria* dans la voie gastro-intestinale de nourrisson non ou partiellement nourris au sein.
